# EUROPÄISCHE PATENTSCHRIFT

(11) **EP 2 276 749 B1**
(45) Veröffentlichungstag und Bekanntmachung des Hinweises auf die Patenterteilung: **18.05.2016**
(21) Anmeldenummer: 09727722.2
(22) Anmeldetag: 27.03.2009
(51) Int. Cl.: C07D 251/18, C07D 251/70, C07C 273/18, C07C 275/10, C08G 12/12, C08G 12/32

(54) **VERFAHREN ZUR HERSTELLUNG EINER VERBINDUNG MIT MINDESTENS EINER ZUMINDEST EINFACH SUBSTITUIERTEN AMINOGRUPPE**
METHOD FOR PRODUCING A COMPOUND WITH AT LEAST ONE AT LEAST MONOSUBSTITUTED AMINO GROUP
PROCÉDÉ DE PRÉPARATION D'UN COMPOSÉ AYANT AU MOINS UN GROUPE AMINO AU MOINS MONOSUBSTITUÉ

(30) Priorität: 31.03.2008 DE 102008016967
(43) Veröffentlichungstag der Anmeldung: 26.01.2011
(73) Patentinhaber: Borealis Agrolinz Melamine GmbH, 4021 Linz (AT)
(72) Erfinder: DICKE, Rene, A-4060 Leonding (AT); BURGER, Martin, 84032 Altdorf (DE)
(74) Vertreter: Maikowski & Ninnemann
(86) Internationale Anmeldenummer: PCT/EP2009/002439
(87) Internationale Veröffentlichungsnummer: WO 2009/121607

(56) Entgegenhaltungen:
- EP-A1- 0 882 720
- EP-A1- 1 057 821
- EP-A2- 0 135 832
- US-A- 4 668 785
- MAGERRAMOV A M ET AL: "Synthesis of substituted ureas from urea and halohydrins" RUSSIAN JOURNAL OF APPLIED CHEMISTRY, NAUKA/INTERPERIODICA, MO, Bd. 77, Nr. 10, 1. Oktober 2004 (2004-10-01), Seiten 1667-1669, XP019299225 ISSN: 1608-3296

## Beschreibung

Die Erfindung betrifft ein Verfahren zur Herstellung einer Verbindung mit mindestens einer zumindest einfach substituierten Aminogruppe gemäß Anspruch 1.

Die JP 2000-063369 A beschreibt ein Verfahren zur Alkylierung von Melamin, in dem Melamin mit einem Alkohol bei hohen Temperaturen in Gegenwart eines Metallkatalysators auf einem mikroporösen Träger umgesetzt wird. Dabei werden vor allem niedrig alkylierte Produkte hergestellt. Bei diesem Verfahren wird einerseits eine niedrige Selektivität der alkylierten Produkte und andererseits die Bildung von Cyanursäureestern als Nebenprodukten durch Hydrolyse bzw. Substitution der Aminogruppen beobachtet. Die Reaktion erfolgt unter einer Stickstoff-, Argon-, Wasserstoff- oder Kohlenmonoxidatmosphäre.

Die EP 0711 760 A1 beschreibt eine Alkylierung von Melamin durch Umsetzung von Melamin in Gegenwart eines Katalysators und einer Atmosphäre aus Argon, Stickstoff, Kohlenmonoxid oder einer Mischung aus Wasserstoff und Kohlenmonoxid. Es werden kein vollständiger Umsatz der Edukte und keine Selektivität bezüglich einzelner Produkte erreicht.

Die EP 1 057 821 A1 beschreibt die Alkylierung von Melamin mit Alkoholen in Gegenwart von Katalysatoren und einer Stickstoff- oder Wassersoffatmosphäre. Es werden kein vollständiger Umsatz der Edukte und keine Selektivität bezüglich einzelner Produkte erreicht.

Shinoda et al. (Appl. Catalysis A: General 194-195 (2000), 375-381) beschreiben eine Methylierung von Melamin ausgehend von Methanol. Für die Katalyse wird ein Metall mit einem sauren Träger benutzt und die Reaktion wird unter Schutzgas- (Argon-) oder Wasserstoffatmosphäre durchgeführt. Die Reaktion erreicht zwar einen vollständigen Umsatz, jedoch nur nach sehr langen Reaktionszeiten. Das gebildete Produktspektrum enthält verschieden substituierte Methylmelamine.
Die EP 0 135 832 beschreibt die Alkylierung von Harnstoff mit Ethanolamin
Der Erfindung liegt die Aufgabe zugrunde, Verbindungen. mit zumindest einer einfach substituierten Aminogruppe bereitzustellen, wobei eine weitere Reaktion der gebildeten Verbindungen auch am Substituenten möglich sein soll.

Diese Aufgabe wird mit einem Verfahren mit den Merkmalen des Anspruchs 1 gelöst. Demnach wird eine Ausgangssubstanz, die mindestens eine Aminogruppe trägt, mit einem durch eine Hydroxylgruppe und durch mindestens eine weitere funktionelle Gruppe substituierten Reagens umgesetzt. Die weitere funktionelle Gruppe ist ausgewählt aus der Klasse umfassend Hydroxylgruppen, Mercaptogruppen, Carbonsäuregruppen, Carbonsäureestergruppen, Carbonsäureamidgruppen, Ketogruppen, Carbonatgruppen, Halogene, Epoxygruppen und Carbamatgruppen. Ist mehr als eine weitere funktionelle Gruppe im Reagens enthalten, sind die einzelnen weiteren funktionellen Gruppen unabhängig voneinander aus der genannten Klasse ausgewählt. Die Ausgangssubstanz und das Reagens sind (ggf. ausschließlicher) Teil einer Reaktionsmischung.

In einer Variante umfasst die Klasse, aus der die funktionelle Gruppe ausgewählt ist, nur Hydroxylgruppen. In einer weiteren Variante umfasst die Klasse, aus der die funktionelle Gruppe ausgewählt ist, nur Mercaptogruppen. In einer weiteren Variante umfasst die Klasse, aus der die funktionelle Gruppe ausgewählt ist, nur Carbonsäuregruppen. In einer weiteren Variante umfasst die Klasse, aus der die funktionelle Gruppe ausgewählt ist, nur Carbonsäureestergruppen. In einer weiteren Variante umfasst die Klasse, aus der die funktionelle Gruppe ausgewählt ist, nur Carbonsäureamidgruppen. In einer weiteren Variante umfasst die Klasse, aus der die funktionelle Gruppe ausgewählt ist, nur Ketogruppen. In einer weiteren Variante umfasst die Klasse, aus der die funktionelle Gruppe ausgewählt ist, nur Carbonatgruppen.

In einer weiteren Variante umfasst die Klasse, aus der die funktionelle Gruppe ausgewählt ist, nur Epoxygruppen. In einer weiteren Variante umfasst die Klasse, aus der die funktionelle Gruppe ausgewählt ist, nur Carbamatgruppen. In einer weiteren Variante umfasst die Klasse, aus der die funktionelle Gruppe ausgewählt ist, eine beliebige Kombination der zuvor erwähnten Gruppen.

Die Aminogruppe der Ausgangssubstanz weist zumindest ein direkt an den Stickstoff gebundenes Wasserstoffatom auf. Das heißt, diese Aminogruppe kann unsubstituiert oder einfach substituiert sein. Nach der Umsetzung mit dem Reagens R-OH ist der Rest R statt des ursprünglichen Wasserstoffatoms an das Stickstoffatom der Aminogruppe gebunden. Mit anderen Worten ist das erfindungsgemäße Verfahren ein Verfahren zur Derivatisierung einer Aminogruppe.

Überraschenderweise hat sich gezeigt, dass eine katalytische Derivatisierung, insbesondere Alkylierung mit substituierten Alkylresten, der Aminogruppe mit einem zumindest eine Hydroxylgruppe tragenden Reagens möglich ist, selbst wenn das Reagens weitere funktionelle Gruppen trägt. Bei Einführung mehrerer Substituenten in die jeweilige Ausgangssubstanz wird zudem eine hohe Selektivität bezüglich der Verteilung der Substituenten an die einzelnen Aminogruppen der Ausgangssubstanz erreicht.

In einer Variante erfolgt die Reaktion in Gegenwart einer weiteren Substanz, die ausgewählt ist aus der Klasse umfassend Ammoniak, Stickstoff, Argon, Wasserstoff und Kohlenmonoxid, so dass insbesondere die Gasphase des Reaktionsansatzes diese weitere Substanz in gasförmiger Form auf. Auch die weitere Substanz kann, beispielsweise in gelöster Form, Teil der Reaktionsmischung sein. Die Reaktionsmischung kann beispielsweise eine Flüssigkeit, eine Dispersion oder eine Suspension sein.

Das Stoffmengenverhältnis zwischen der weiteren Substanz und dem Reagens beträgt in einer Variante 0,1 bis 2, insbesondere 0,5 bis 1,5 und ganz besonders 0,8 bis 1,3.

Ammoniak ist besonders als weitere Substanz geeignet, da sich gezeigt hat, dass der Einsatz von Ammoniak als Gas oder als Lösung zu einer nahezu vollständigen Unterdrückung von Nebenprodukten führt und dass gleichzeitig die Selektivität der Derivatisierungsreaktion signifikant ansteigt. Insbesondere kann die Nebenproduktbildung durch Hydrolyse signifikant reduziert oder unterdrückt werden.

Die Umsetzung erfolgt in einer Ausgestaltung bei einem Gesamtdruck von ca. 1 bis ca. 200 bar, insbesondere bei ca. 40 bar bis ca. 180 bar und ganz besonders bei ca. 60 bar bis ca. 140 bar. Der Gesamtdruck setzt sich dabei aus den Partialdrücken der in der Gasphase enthaltenen Gase zusammen. Die weitere Substanz kann dabei zumindest einen Teil der in der Gasphase enthaltenen Gase darstellen. Auch das Reagens kann zumindest teilweise die in der Gasphase enthaltenen Gase ausmachen.

Die Umsetzung erfolgt unter Einwirkung eines Katalysators, um die Umsatzraten zu erhöhen, die Reaktionstemperaturen auf einem niedrigeren Niveau halten zu können, die Selektivität bezüglich der Produkte zu erhöhen und/oder die Reaktionszeiten zu verkürzen.

Der Katalysator weist ein Metall oder ein Metalloxid auf. Auch Mischungen verschiedener Metalle und/oder Metalloxide sind möglich.

Insbesondere weist der Katalysator ein Metall aus der 8., 9. oder 10. IUPAC-Gruppe (VIII. Nebengruppe) des Periodensystems auf. Dazu gehören unter anderem Eisen, Cobalt, Nickel, Ruthenium, Rhodium, Palladium und Platin.

Die eingesetzte Menge des Katalysators liegt in einer Variante im Bereich von 0,001 bis 20 Mol-%, insbesondere 0,01 bis 10 Mol-%, insbesondere 0,1 bis 1 Mol-% und ganz besonders 0,1 bis 0,5 Mol-%, jeweils bezogen auf die Stoffmenge der umzusetzenden Verbindung.

In einer weiteren Ausgestaltung weist der Katalysator ein Trägermaterial auf. Wird beispielsweise ein poröses Trägermaterial verwendet, lässt sich die Oberfläche des Katalysators erhöhen und die Menge des katalytisch aktiven Metalls oder Metalloxids verringern.

Geignete Trägermaterialien sind beispielsweise Zeolithe, Alumosilikate, Alumophosphate, Metalloxide, Silikate, Schichtsilikate, Aluminiumoxid, Siliziumdioxid sowie Kohlenstoff.

Beispiele für Zeolithe sind Beta-Zeolith (BEA), Y-Zeolith, Faujasit, Mordenit, ZSM-5, Zeolith X, Zeolith A.

Beispiele für Schichtsilikate sind Montmorillonit, Mordenit, Bentonit, Kaolinit, Muskovit, Hectorit, Fluorhectorit, Kanemit, Revdit, Grumantit, Ilerit, Saponit, Beidelit, Nontronit, Neue Seite

Stevensit, Laponit, Taneolit, Vermiculit, Halloysit, Volkonskoit, Magadit, Rectorit, Kenyait, Sauconit, Borfluorphlogopite und/oder synthetische Smectite.

Die Katalysatoren können als Pulver-, Formkörper- und Monolithkatalysatoren, letztere beispielsweise auf Wabenkörpern, eingesetzt werden. Für die Umsetzung kommen beispielsweise Festbettreaktoren, Wirbelschichtreaktoren, Rührkesselreaktoren oder Rohrreaktoren zum Einsatz. Die Reaktoren werden in einer Variante für die Umsetzung bei Temperaturen von ca. 100 °C bis ca. 300 °C, insbesondere ca. 150 °C bis ca. 250 °C und ganz besonders ca. 180 °C bis ca. 240 °C betrieben.

Die notwendigen Reaktionszeiten liegen in einer Ausgestaltung unter 100 Stunden, insbesondere zwischen ca. 1 Minute und ca. 50 Stunden, insbesondere zwischen ca. 1 Stunde und ca. 20 Stunden, und ganz besonders zwischen ca. 4 Stunden und ca. 12 Stunden.

In einer alternativen Ausgestaltung wird der Katalysator nach erfolgter Umsetzung, das heißt nachdem die Reaktion beendet oder abgebrochen wird, beispielsweise durch Filtration von der Reaktionsmischung getrennt und weiter verarbeitet.

Die Ausgangssubstanz ist ein Harnstoff bzw. Harnstoffderivat der allgemeinen Formel (II): 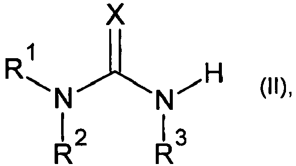 wobei
- R¹, R², R³ unabhängig voneinander H, lineares oder verzweigtes C₁₋C₂₀-Alkyl, C₅-C₂₀-Cycloalkyl, C₅-C₂₀-Aryl, C₁-C₂₀-alkylsubstituiertes C₅-C₂₀-Aryl, das jeweils durch ein oder mehrere Sauerstoffatome, Schwefelatome und/oder substituierte Stickstoffatome und/oder durch eine oder mehrere Gruppen des Typs -C(O)O-, -OC(O)-, -C(O)- und/oder -OC(O)O- unterbrochen sein kann und/oder durch eine oder mehrere Hydroxygruppen und/oder Mercaptogruppen funktionalisiert sein kann, bedeuten und
- X O oder S bedeutet.
   Als Reagens wird eine Verbindung mit der allgemeinen Formel R¹⁰-OH verwendet, so dass mindestens eine Hydroxylgruppe als erste funktionelle Gruppe im Reagens enthalten ist, wobei
- R¹⁰ lineares oder verzweigtes C₁-C₂₀-Alkyl, C₅-C₂₀-Cycloalkyl, oder C₁,-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein kann und mindestens eine weitere funktionelle Gruppe aufweist, die ausgewählt ist aus der Klasse umfassend Hydroxylgruppen, Mercaptogruppen, Carbonsäuregruppen, Carbonsäureestergruppen, Carbonsäureamidgruppen, Ketogruppen, Carbonatgruppen, Epoxygruppen und Carbamatgruppen, bedeutet.

In einer Variante ist mindestens eine Hydroxylgruppe des Reagens am Alkylrest und nicht am Arylrest gebunden, wenn R¹⁰ ein C₁-C₂₀-alkylsubstituiertes C₅-C₂₀-Aryl, welches wie zuvor unterbrochen oder substituiert sein kann, bedeutet. Dabei kann es auch vorgesehen sein, dass sämtliche Hydroxylgruppen am Alkylrest und nicht am Arylrest gebunden sind. Auf diese Weise lässt sich die Bildung arylierter Triazin- oder Harnstoffderivate zugunsten der Bildung arylsubstituierter alkylierter Derivate vermeiden.

Beispiele für Harnstoffderivate als Ausgangssubstanz sind Hydroxyethylharnstoff und Ethylenharnstoff. Auch nicht derivatisierter Harnstoff kann als Ausgangssubstanz eingesetzt werden.

Als Reagens sind beispielsweise Polyalkohole (worunter auch Diole und Oligoole zu verstehen sind), Thiole und andere Hydroxyverbindungen mit mindestens einer weiteren funktionellen Gruppe geeignet.

Beispiele für geeignete Polyalkohole sind Ethylenglykol, Diethylenglykol, Glycerol, Trimethylolpropan, Pentaerythrit, Tripropylenglykol, Trisopropanolamin, Triethanolamin, Hexandiol, Butandiol und Glycerolmonostearat.

Beispiele für geeignete Thiole sind Mercaptoethanol, Mercaptopropanol, Mercaptomethylbutanol und Mercaptohexanol.

Beispiele für Hydroxyverbindungen mit mindestens einer weiteren funktionellen Gruppen sind Hydroxyessigsäure, Hydroxyessigsäuremethylester, Hydroxyessigsäureethylester, 2,2-Bis(hydroxymethyl)propionsäure, Methyl-2,2-dimethyl-3-hydroxypropionat, *tert*-Butyl-3-hydroxypropionat und *N,N*-Diethyl-2-hydroxyacetamid.

In einer Variante des Verfahrens reicht es bei kurzkettigen Alkoholen als Edukten für die Derivatisierung der Ausgangssubstanz aus, den Alkohol im Überschuss einzusetzen, um diesen auch als Lösungsmittel zu nutzen. In einer weiteren Variante werden bei Verwendung von längerkettigen Alkoholen (mehr als 8, 10 oder 12 C-Atome) inerte Lösungsmittel als Lösungsvermittler eingesetzt, um eine bessere Umsetzung zu erreichen. Grundsätzlich können auch Lösungsvermittler bei kurzkettigeren Alkoholen eingesetzt werden, wenn diese beispielsweise stark verzweigt sind und daher eine höhere Viskosität aufweisen. Der Einsatz eines Lösungsvermittlers ist immer dann angebracht, wenn die Mischung ohne Lösungsvermittler nicht mehr rührfähig ist.

Beispiele für derartige Lösungsvermittler sind Tetrahydrofuran, Diethylether, Dimethoxymethan, Dimethoxyethan, Diethoxymethan, Diethoxyethan, Ethylenglykoldiethylether, Ethylenglykoldibutylether, Diethylenglykoldiethylether, Dioxan, Benzen, Toluen, Xylen, Mesitylen, Cumen, Chlorbenzen, Pentan, Hexan, Cyclohexan, Heptan, Octan, Acetonitril, Methylacetat, Ethylacetat, Methylbenzoat, N,N-Dimethylformamid, N,N-Dimethylacetamid, N-Methylpyrrolidon, 1,3-Dimethylimidazolidinon.

Bei einem Reagens mit mehreren Hydroxylgruppen empfiehlt es sich, in starker Verdünnung zu arbeiten, um Nebenreaktionen, durch die oligomere oder polymere Verbindungen aus mehreren Triazin- oder Harnstoffeinheiten gebildet werden, zu vermeiden. Beispielsweise können sich, wenn in hoher Konzentration gearbeitet und Glykol (1,2-Ethandiol) als Reagens verwendet wird, Moleküle aus zwei Triazineinheiten bilden, bei denen die beiden Triazineinheiten über eine Ethylenbrücke miteinander verbunden sind. Dies ist unerwünscht und kann durch Arbeiten bei niedriger Verdünnung vermieden werden.

Die gebildete Verbindung ist ein Harnstoffderivat der allgemeinen Formel (IV): 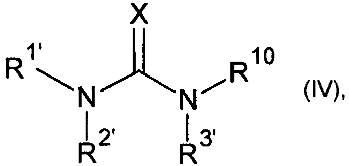 wobei
- R^{1'}, R^{2'}, R^{3'} unabhängig voneinander
   - R¹⁰,
   - eine an einen am selben Stickstoffatom des Triazinderivats oder des Harnstoffderivats gebundenen Rest R¹⁰ gebundene kovalente Bindung, so dass sich eine zyklische Struktur aus dem Stickstoffatom und dem Rest R¹⁰ ausbildet,
   - H oder
   - lineares oder verzweigtes C₁-C₂₀-Alkyl, C₅-C₂₀-Cycloalkyl, C₅-C₂₀-Aryl, C₁-C₂₀-alkylsubstituiertes C₅-C₂₀-Aryl, das jeweils durch ein oder mehrere Sauerstoffatome, Schwefelatome und/oder substituierte Stickstoffatome und/oder durch eine oder mehrere Gruppen des Typs -C(O)O-, -OC(O)-, -C(O)- und/oder -OC(O)O- unterbrochen sein kann und/oder durch eine oder mehrere Hydroxygruppen und/oder Mercaptogruppen funktionalisiert sein kann, bedeuten,
- R¹⁰ lineares oder verzweigtes C₁-C₂₀-Alkyl, C₅-C₂₀-Cycloalkyl oder C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein kann, bedeutet und
   a) mindestens eine weitere funktionelle Gruppe aufweist, die ausgewählt ist aus der Klasse umfassend Hydroxylgruppen, Mercaptogruppen, Carbonsäuregruppen, Carbonsäureestergruppen, Carbonsäureamidgruppen, Ketogruppen, Carbonatgruppen, Halogene, Epoxygruppen und Carbamatgruppen, oder
   b) dabei eine zyklische Struktur mit zwei an dasselbe Stickstoffatom der Verbindungen der allgemeinen Formeln (III) oder (IV) gebundenen kovalenten Bindungen ausbildet, wobei eine der beiden kovalenten Bindungen durch einen der Reste **R^{1'}, R^{2'}, R^{3'}** bereitgestellt wird und wobei im Rest R¹⁰ mindestens eine weitere funktionelle Gruppe vorhanden sein kann, die ausgewählt ist aus der Klasse umfassend Hydroxylgruppen, Mercaptogruppen, Carbonsäuregruppen, Carbonsäureestergruppen, Carbonsäureamidgruppen, Ketogruppen, Carbonatgruppen, Halogene, Epoxygruppen und Carbamatgruppen, und
- X O oder S bedeutet.

Der Begriff "weitere funktionelle Gruppe" in Bezug auf die Bedeutung des Rests **R¹⁰** in einer Verbindung der allgemeinen Formel (III) oder (IV) ist dabei so zu verstehen, dass im Rest **R¹⁰** zumindest eine funktionelle Gruppe aus der genannten Klasse vorhanden ist oder sein kann, ohne dass zwingenderweise eine andere funktionelle Gruppe in diesem Rest vorhanden ist. Die ursprünglich im Rest **R¹⁰** vorhandene Hydroxylgruppe ist nach der erfolgten Reaktion mit dem Triazinderivat der allgemeinen Formel (I) oder dem Harnstoff bzw. Harnstoffderivat der allgemeinen Formel (II) zu einer Verbindung der allgemeinen Formel (III) oder (IV) nicht mehr vorhanden.

Eine zyklische Struktur gemäß Variante b) bildet sich beispielsweise dann, wenn ein Reagens eingesetzt wird, das mindestens zwei Hydroxylgruppen trägt, die jeweils mit demselben Stickstoffatom der Ausgangsverbindung reagieren.

In einer Variante des Verfahrens kommt mindestens einem Rest, insbesondere mindestens zwei Resten, insbesondere mindestens drei Resten, der Reste **R^{1'}, R^{2'} R^{3'},** nur die Bedeutung des Rests **R¹⁰** und insbesondere nicht die Bedeutung H zu. Dies ist insbesondere dann der Fall, wenn die entsprechenden Reste **R^{1'}, R^{2'}, R^{3'},** in der Ausgangssubstanz einen Wasserstoffrest bedeutet haben. Mit anderen Worten können unterschiedlich viele Aminogruppen der gebildeten Verbindung (beispielsweise eine, zwei oder drei Aminogruppen) voneinander unabhängig unterschiedlich hoch (beispielsweise einfach oder zweifach) mit dem Rest R¹⁰ substituiert sein.

Durch geeignete Versuchsparameter können im Wesentlichen reine N,N'-Dihydroxyalkylverbindungen, N,N',N"-Trihydroxyalkylverbindungen, N,N',N"-Trihydroxyalkyl-N,N',N"-trialkylverbindungen, N,N,N',N"-Tetrahydroxyalkylverbindungen oder N,N,N',N',N",N"-Hexahydroxyalkylverbindungen hergestellt werden.

Durch geeignete Versuchsparameter können auch im Wesentlichen reine N,N'-Dicarbonylalkylverbindungen, N,N',N"-Tricarbonylalkylverbindungen, N,N',N"-Tricarbonylalkyl-N,N',N"-trialkylverbindungen, N,N,N',N"-Tetracarbonylalkylverbindungen oder N,N,N',N',N",N"-Hexacarbonylalkylverbindungen hergestellt werden.

In einer Ausgestaltung ist die gebildete Verbindung ausgewählt aus der Gruppe umfassend Harnstoff-N-mono(alkylcarbonsäure), Harnstoff-N,N'-di(alkylcarbonsäure), Harnstoff-N,N,N'-tri(alkylcarbonsäure), Harnstoff-N,N,N',N'-tetra(alkylcarbonsäure).

Als Alkylrest kommen dabei jeweils insbesondere Methyl-, Ethyl-, Butyl und/oder Hexylreste (oder deren Mischungen) in Betracht, aber auch alle anderen für den Rest R¹⁰ oben angegeben Bedeutungen. Als Hydroxyalkylrest kommen alle Hydroxygruppen tragenden Reste R¹⁰, beispielsweise 2-Hydroxyethyl-, Hydroxypropyl- und/oder Hydroxyethoxyethylreste, in Betracht.

Anhand der nachfolgenden Reaktionsgleichungen soll eine beispielhafte Ausgestaltung des beanspruchten Verfahrens näher erläutert werden. Dabei bedeuten "T" eine gegenüber der Raumtemperatur erhöhte Temperatur und "p" einen gegenüber dem Standardluftdruck erhöhten Druck (besondere Parameterausgestaltungen bzw. Reaktionsbedingungen und Bedeutungen der Reste Rⁿ sind weiter oben erläutert):

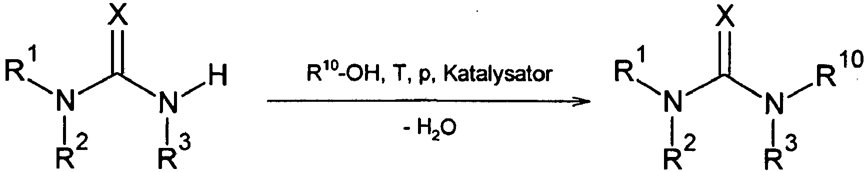

Durch die Umsetzung der Ausgangssubstanz wird ein an den Stickstoff einer Aminogruppe gebundenes Wasserstoffatom durch den Rest R¹⁰ aus dem eingesetzten Reagens ersetzt; es erfolgt eine Derivatisierung der Aminogruppe. Nach Abschluss der Reaktion weist die derivatisierte Aminogruppe zumindest einen von einem Wasserstoffatom verschiedenen Substituenten, nämlich R¹⁰, auf. Je nach Reaktionsbedingungen können auch weitere Reste Rⁿ, die zuvor ein Wasserstoffatom darstellten, durch den Rest R¹⁰ ersetzt werden. Auf diese Weise lassen sich Verbindungen mit unterschiedlich stark substituierten Aminogruppen herstellen.

Durch die erzielte Reinheit eignen sich die nach diesem Verfahren hergestellten derivatisierten Verbindungen wie beispielsweise derivatisierte Aminotriazine und derivatisierte Harnstoffe für die Verwendung als Formaldehydharze. Unter dem Begriff "Formatdehydharz" wird dabei ein Harz aus Formaldehyd und der entsprechenden gebildeten Verbindung verstanden. Diese Formaldehydharze weisen spezielle Eigenschaften hinsichtlich Rheologie, Hydrophilie bzw. Lipophilie und Oberflächeneigenschaften auf. Sie eignen sich insbesondere zur Verwendung im Bereich der Laminatbeschichtung der holzverarbeitenden Industrie.

Insbesondere alkylierte Verbindungen eignen sich auch als Vernetzer. Weitere Einsatzgebiete der gebildeten Verbindungen, insbesondere der hydroxyalkylierten Verbindungen wie beispielsweise den hydroxyalkylierten Harnstoffen, sind der Bereich der Additive zur Plastifizierung, der Bereich der Flammschutzadditive, der Bereich der Comonomere für ein Polyurethan und der Bereich der Agrochemikalien.

Wenn längerkettige lipophile Reste wie beispielsweise Alkylreste an die eine Aminogruppe tragende Ausgangssubstanz gebunden sind, ist die Hydrophobizität der gebildeten Verbindung höher als die der Ausgangssubstanz. Weist die weitere funktionelle Gruppe in den einzuführenden Alkylresten zusätzlich eine Polarität auf, kann die Hydrophilie der gebildeten Verbindung (zumindest partiell lokal) gegenüber der Ausgangsverbindung jedoch erhöht sein. Dann eignen sich die mit dem beanspruchten Verfahren hergestellten Verbindungen besonders vorteilhaft dazu, in einer Mischung mit einem Polyolefin, insbesondere einem Polyethylen (Polyethen) oder Polypropylen (Polypropen), eingesetzt zu werden, da sie eine günstigere Wechselwirkung des Polyolefins mit zu kontaktierenden Oberflächen oder anderen Substanzen ermöglichen. Auf diese Weise lassen sich beispielsweise die Flammschutzeigenschaften oder Oberflächeneigenschaften (in Bezug auf eine verbesserte Haftung gegenüber einer aufzutragenden Beschichtung) eines aus der Mischung des Polyolefins und der gebildeten Verbindung bestehenden Gegenstands gegenüber den eines aus einem unmodifizierten Polyolefin bestehenden Gegenstands verbessern.

Es ist auch möglich, einen Alkohol mit mindestens drei Kohlenstoffatomen in eine der Ausgangsverbindungen einzuführen und durch anschließende Wassereliminierung eine Doppelbindung im Substituenten der gebildeten Verbindung zu schaffen, die dann an ein Polyolefin gepfropft werden kann.

Weitere Details der Erfindung werden anhand des nachfolgenden Beispiels näher erläutert, Sofern nicht explizit etwas anderes angegeben wird, sind alle Prozentangaben in den Beispielen wie auch in den übrigen Teilen der Beschreibung und den Ansprüchen als Massenprozent zu verstehen.

### Beispiel:

In einem 500ml Rührautoklav werden 3,0 g Harnstoff, 150 g Ethylenglykol und 5 g eines Ru/BEA Katalysators intensiv gemischt, damit der Katalysator nicht zu Boden sinkt. Nach Verschließen des Autoklaven wird auf 200°C aufgeheizt. Nach 4 Stunden Reaktionszeit wird die Reaktion abgebrochen und der Autoklav abgekühlt und entspannt. Die erkaltete Lösung wird über einen Filter vom Katalysator getrennt und im Hochvakuum eingeengt. Auf diese Weise wurden 3,5 g Produkt isoliert. Eine Bestimmung der Zusammensetzung des Produkts mittels quantitativer HPLC ergab 76% N,N'-Di-(hydroxyethyl)-harnstoff, 20% N-Mono-(hydroxyethyl)-harnstoff und 4% N,N,N'-Tri-(hydroxyethyl)-harnstoff.

## Patentansprüche

1. Verfahren zur Herstellung einer Verbindung mit mindestens einer zumindest einfach substituierten Aminogruppe, **dadurch gekennzeichnet, dass**
- eine mindestens eine Aminogruppe tragende Ausgangssubstanz in Form eines Harnstoffs bzw. Harnstoffderivates der allgemeinen Formel (II) 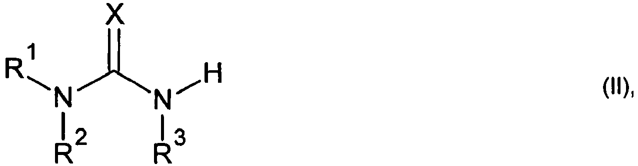 wobei
• **R¹, R², R³,** unabhängig voneinander H, lineares oder verzweigtes C₁C₂₀-Alkyl, C₅-C₂₀-Cycloalkyl, C₅-C₂₀-Ary). C₁-C₂₀-alkylsubstituiertes C₅-C₂₀-Aryl, das jeweils durch ein oder mehrere Sauerstoffatome, Schwefelatome und/oder substituierte Stickstoffatome und/oder durch eine oder mehrere Gruppen des Typs -C(O)O-, -OC(O)-, -C(O)- und/oder -OC(O)O- unterbrochen sein kann und/oder durch eine oder mehrere Hydroxygruppen und/oder Mercaptogruppen funktionalisiert sein kann, bedeuten und
• X O oder S bedeutet,
- mit einem eine erste funktionelle Gruppe und mindestens eine weitere funktionelle Gruppe tragenden Reagens in einer Reaktionsmischung umgesetzt wird, wobei das Reagens die allgemeine Formel R¹⁰-OH aufweist, so dass mindestens eine Hydroxylgruppe als erste funktionelle Gruppe im Reagens enthalten ist, wobei
**R¹⁰** lineares oder verzweigtes C₁-C₂₀-Alkyl, C₅-C₂₀-Cycloalkyl, oder C₁-C₂₀-alkylsubstituiertes C₅-C₂₀-Aryl, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O- unterbrochen sein kann und mindestens eine weitere funktionelle Gruppe aufweist, die ausgewählt ist Neue Ansprüche_3. Bescheid vom 30.4.2015
aus der Klasse umfassend Hydroxylgruppen, Mercaptogruppen, Carbonsäuregruppen, Carbonsäureestergruppen, Carbonsäureamidgruppen, Ketogruppen, Carbonatgruppen, Epoxygruppen und Carbamatgruppen, bedeutet.
wobei
die Reaktion in Gegenwart eines Katalysators in Form eines Metalls aus der 8., 9. oder 10. Gruppe des Periodensystems und/oder dessen Metalloxid erfolgt, wobei die gebildete Verbindung ein Harnstoffderivat der allgemeinen Formel (IV) ist: 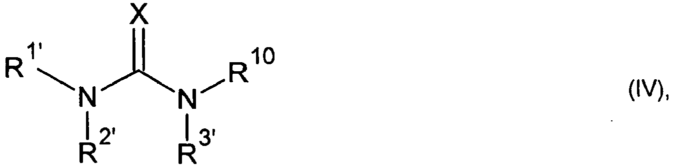 wobei
• R^{1'}, R^{2'}, R^{3'}, unabhängig voneinander
- **R¹⁰,**
- eine an einen am selben Stickstoffatom des Harnstoffderivats gebundenen Rest **R¹⁰** gebundene kovalente Bindung, so dass sich eine zyklische Struktur aus dem Stickstoffatom und dem Rest **R¹⁰** ausbildet,
- H oder
- lineares oder verzweigtes C₁-C₂₀-Alkyl, C₅-C₂₀-Cycloalkyl, C₅-C₂₀-Aryl, C₁-C₂₀-alkylsubstituiertes C₅-C₂₀-Aryl, das jeweils durch ein oder mehrere Sauerstoffatome, Schwefelatome und/oder substituierte Stickstoffatome und/oder durch eine oder mehrere Gruppen des Typs -C(O)O-, -OC(O)-, -C(O)- und/oder -OC(O)O- unterbrochen sein kann und/oder durch eine oder mehrere Hydroxygruppen und/oder Mercaptogruppen funktionalisiert sein kann, bedeuten,
• **R¹⁰** lineares oder verzweigtes C₁-C₂₀-Alkyl, C₅-C₂₀-Cycloalkyl oder C₁-C₂₀-alkylsubstituiertes C₅-C₂₀-Aryl bedeutet, das jeweils durch ein oder mehrere Sauerstoffatome, Schwefelatome, substituierte Stickstoffatome und/oder durch eine oder mehrere Gruppen des Typs -C(O)O-, -OC(O)-, -C(O)- und/oder -OC(O)O-unterbrochen sein kann und
a) mindestens eine weitere funktionelle Gruppe aufweist, die ausgewählt ist aus der Klasse umfassend Hydroxylgruppen, Mercaptogruppen, Carbonsäuregruppen, Carbonsäureestergruppen, Carbonsäureamidgruppen, Ketogruppen, Carbonatgruppen, Epoxygruppen und Carbamatgruppen, oder
b) dabei eine zyklische Struktur mit zwei an dasselbe Stickstoffatom der Verbindungen der allgemeinen Formeln (III) oder (IV) gebundenen kovalenten Bindungen ausbildet, wobei eine der beiden kovalenten Bindungen durch einen der Reste **R^{1'}, R^{2'}, R^{3'}** bereitgestellt wird und wobei im Rest R^{10'} mindestens eine weitere funktionelle Gruppe vorhanden sein kann, die ausgewählt ist aus der Klasse umfassend Hydroxylgruppen, Mercaptogruppen, Carbonsäuregruppen, Carbonsäureestergruppen, Carbonsäureamidgruppen, Ketogruppen, Carbonatgruppen, Epoxygruppen und Carbamatgruppen, und
• X O oder S bedeutet.

2. Verfahren nach Anspruch 1, **dadurch gekennzeichnet, dass** die Reaktion in Gegenwart mindestens einer weiteren Substanz erfolgt, die ausgewählt ist aus der Klasse umfassend Ammoniak, Stickstoff, Argon, Wasserstoff und/oder Kohlenmonoxid.

3. Verfahren nach Anspruch 2, **dadurch gekennzeichnet, dass** das Stoffmengenverhältnis von der weiteren Substanz zu dem Reagens 0,1 bis 2, insbesondere 0,5 bis 1,5 und ganz besonders 0,8 bis 1,3 beträgt.

4. Verfahren nach einem der vorhergehenden Ansprüche, **dadurch gekennzeichnet, dass** die Umsetzung bei einem Gesamtdruck von 1 bis 200 bar, insbesondere 40 bis 180 bar und ganz besonders 60 bis 140 bar und bei einer Temperatur von 100°C bis 300 °C, insbesondere 150 °C bis 250°C, insbesondere 180°C bis 240 °C erfolgt.

5. Verfahren nach einem der vorhergehenden Ansprüche, **dadurch gekennzeichnet, dass** die Umsetzung während einer Reaktionsdauer von 1 Minute bis 100 Stunden, insbesondere 1 Stunde bis 20 Stunden und ganz besonders 4 Stunden bis 12 Stunden erfolgt.

6. Verfahren nach einem der vorhergehenden Ansprüche, **dadurch gekennzeichnet, dass** der Katalysator ein Trägermaterial aufweist.

7. Verfahren nach Anspruch 6, **dadurch gekennzeichnet, dass** das Trägermaterial ein Zeolith, ein Alumosilikat, ein Alumophosphat, ein Metalloxid, ein Silikat, ein Schichtsilikat, Aluminiumoxid, Siliziumdioxid und/oder Kohlenstoff aufweist.

8. Verfahren nach einem der vorhergehenden Ansprüche, **dadurch gekennzeichnet, dass** mindestens eine Hydroxylgruppe des Reagens am Alkylrest und nicht am Arylrest vorliegt, wenn **R¹⁰** ein C₁-C₂₀-alkylsubstituiertes C₅-C₂₀-Aryl bedeutet.

9. Verfahren nach Anspruch einem der vorhergehenden Ansprüche, **dadurch gekennzeichnet, dass** mindestens einem Rest, insbesondere mindestens zwei Resten, insbesondere mindestens drei Resten, der Reste **R^{1'}, R^{2'}, R^{3'}** die Bedeutung des Rests **R¹⁰** zukommt.

10. Verfahren nach einem der vorhergehenden Ansprüche, **dadurch gekennzeichnet, dass** die gebildete Verbindung ausgewählt ist aus der Gruppe umfassend Harnstoff-N-mono(alkylcarbonsäure), Harnstoff-N,N'-di(alkylcarbonsäure), Harnstoff-N,N,N'-tri(alkylcarbonsäure), Harnstoff-N,N,N',N'-tetra(alkylcarbonsäure).

## Claims

1. Method for producing a compound having at least one at least monosubstituted amino group, **characterized in that**
- a starting substance having at least one amino group in form of a urea or urea derivative of the general formula (II) 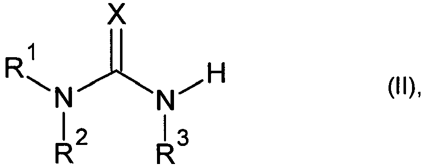 wherein
• R¹, R², R³, mean independently from each other H, linear or branched C₁-C₂₀-alkyl, C₅-C₂₀-cycloalkyl, C₅-C₂₀-aryl, C₁-C₂₀-alkylsubstituted C₅-C₂₀-aryl, which in each case can be interrupted by one or multiple oxygen atoms, sulphur atoms and/or substituted nitrogen atoms and/or by one or multiple groups of the type - C(O)O-, -OC(O)-, -C(O)- and/or -OC(O)O- and/or can be functionalized by one or multiple hydroxyl groups and/or mercapto groups, and
• X means O or S,
- with a reagent having a first functional group and at least one further functional group in a reaction mixture, wherein the reagent has the general formula R¹⁰-OH so that at least one hydroxyl group is present as a first functional group in the reagent, whereat
R¹⁰ means a linear or branched C₁-C₂₀-alkyl, C₅-C₂₀-cycloalkyl or C₁-C₂₀-alkyl substituted C₅-C₂₀-aryl, which in each case can be interrupted by one or multiple oxygen atoms, sulphur atoms, substituted nitrogen atoms and/or by one or multiple groups of the type -C(O)O-, -OC(O)-, -C(O)- and/or -OC(O)O- and has at least one further functional group which is selected from the class comprising hydroxyl groups, mercapto groups, carboxylic acid groups, carboxylic acid ester groups, carboxylic acid amide groups, keto groups, carbonate groups, halogen, epoxy groups and carbamate groups,
wherein
the reaction is carried out in the presence of a catalyst in form of a metal from 8^{th}, 9^{th} or 10^{th} group of the periodic system and/or a metal oxide comprises a metal from 8^{th}, 9^{th} or 10^{th} group of the periodic system, wherein
the produced compound is a urea derivative of the general formula (IV): 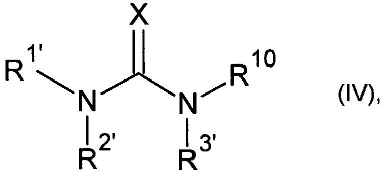 whereby
• R^{1'}, R^{2'}, R^{3'} mean independently from each other,
- R¹⁰,
- a covalent bond bound to the moiety R¹⁰ being bound to the same nitrogen atom of the triazine derivative or urea derivative so that a cyclic structure is formed from the nitrogen atom and the moiety R¹⁰,
- H or
- linear or branched C₁-C₂₀-alkyl, C₅-C₂₀-cycloalkyl, C₅-C₂₀-aryl, C₁-C₂₀-alkylsubstituted C₅-C₂₀-aryl, which in each case can be interrupted by one or multiple oxygen atoms, sulphur atoms and/or substituted nitrogen atoms and/or by one or multiple groups of the type -C(O)O-, -OC(O)-, -C(O)- and/or -OC(O)O-and/or can be functionalized by one or multiple hydroxyl groups and/or mercapto groups,
• R¹⁰ means a linear or branched C₁-C₂₀-alkyl, C₅-C₂₀-cyclo alkyl or C₁-C₂₀-alkylsubstituted C₅-C₂₀-aryl, which in each case can be interrupted by one or multiple oxygen atoms, sulphur atoms, substituted nitrogen atoms and/or by one or multiple groups of the type -C(O)O-, -OC(O)-, -C(O)- and/or -OC(O)O-, and
a) has at least one further functional group, which is selected from the class comprising hydroxyl groups, mercapto groups, carboxylic acid groups, carboxylic acid ester groups, carboxylic acid amide groups, keto groups, carbonate groups, halogens, epoxy groups and carbamate groups. or
b) thereby forms a cyclic structure with two covalent bonds bonded to the same nitrogen atom of the compounds of the general formula (III) or (IV), whereat one of the two covalent bonds is provided by one of the moieties R^{1'}, R^{2'}, R^{3'} and whereat in the moiety R¹⁰ at least one further functional group can be present, which is selected from the class comprising hydroxyl groups, mercapto groups, carboxylic acid groups, carboxylic acid ester groups, carboxylic acid amide groups, keto groups, carbonate groups, halogens, epoxy groups and carbamate groups, and
• X means O or S.

2. Method according to claim 1, **characterized in that** the reaction is carried out in the presence of at least one further substance selected from the class comprising ammonia, nitrogen, argon, hydrogen and carbon monoxide.

3. Method according to claim 2, **characterized in that** the molar ratio of the further substance to the reagent is 0.1 to 2, in particular 0.5 to 1.5 and especially in particular 0.8 to 1.3.

4. Method according to one of the preceding claims, **characterized in that** the reaction occurs at a total pressure of 1 to 200 bar, in particular 40 to 180 bar and especially in particular 60 to 140 bar and the reaction occurs at a temperature of 100°C to 300°C, in particular 150°C to 250°C, in particular 180°C to 240°C.

5. Method according to one of the preceding claims, **characterized in that** the reaction occurs during a reaction time of 1 minute to 100 hours, in particular 1 hour to 20 hours and especially particular 4 hours to 12 hours.

6. Method according to one of the preceding claims, **characterized in that** the catalyst has a carrier material.

7. Method according to claim 6, **characterized in that** the carrier material is a zeolithe, an alumo silicate, an alumo phosphate, a metal oxide, a silicate, a layered silicate, an aluminium oxide, silizium dioxide and/or carbon.

8. Method according to one of the preceding claims, **characterized in that** at least one hydroxyl group of the reagent is present at the alkyl moiety and not at the aryl moiety, if R¹⁰ means a C₁-C₂₀ alkyl substituted C₅-C₂₀-aryl.

9. Method according to one of the preceding claims, **characterized in that** at least one moiety, in particular at least two moieties, in particular at least three moieties R^{1'}, R^{2'}, R^{3'} have the meaning of the moiety R¹⁰.

10. Method according to one of the preceding claims, **characterized in that** the formed compound is selected from the group comprising urea-N-mono(alkylcarboxylic acid), urea-N,N'-di(alkylcarboxylic acid), urea-N,N,N'-tri(alkylcarboxylic acid), urea-N,N,N',N'-tetra(alkylcarboxylic acid).

## Revendications

1. Procédé de préparation d'un composé ayant au moins un groupe amino au moins monosubstitué, **caractérisé en ce que**
- l'on fait réagir une substance de départ comportant au moins un groupe amino sous forme d'urée ou de dérivé d'urée de formule générale (II) 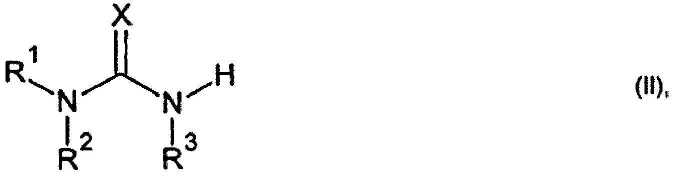 dans laquelle
-- R¹, R², R³, indépendamment les uns des autres, représentent H, un groupe alkyle en C₁ à C₂₀ linéaire ou ramifié, cycloalkyle en C₅ à C₂₀, aryle en C₅ à C₂₀, aryle en C₅ à C₂₀ substitué par un groupe alkyle en C₁ à C₂₀, qui peut être interrompu, chacun, par un ou plusieurs atomes d'oxygène, atomes de soufre, et/ou atomes d'azote substitués, et/ou par un ou plusieurs groupes du type -C(O)O-, -OC(O)-, -C(O)- et/ou
-OC(O)O- et/ou qui peut être fonctionnalisé par un ou plusieurs groupes hydroxy et/ou groupes mercapto, et
-- X représente O ou S,
- avec un réactif comportant un premier groupe fonctionnel et au moins un autre groupe fonctionnel dans un mélange réactionnel, ledit réactif ayant pour formule générale R¹⁰-OH de sorte qu'au moins un groupe hydroxyle soit présent dans le réactif en tant que premier groupe fonctionnel,
R¹⁰ représentant un groupe alkyle en C₁ à C₂₀ linéaire ou ramifié, cycloalkyle en C₅ à C₂₀ ou aryle en C₅ à C₂₀ substitué par un groupe alkyle en C₁ à C₂₀, qui peut être interrompu, chacun, par un ou plusieurs atomes d'oxygène, atomes de soufre, et/ou atomes d'azote substitués, et/ou par un ou plusieurs groupes du type -C(O)O-, - OC(O)-, -C(O)- et/ou -OC(O)O- et comprenant au moins un autre groupe fonctionnel choisi dans la classe comprenant les groupes hydroxyle, les groupes mercapto, les groupes acide carboxylique, les groupes ester d'acide carboxylique, les groupes amide d'acide carboxylique, les groupes cétone, les groupes carbonate, les groupes époxy et les groupes carbamate,
la réaction se faisant en présence d'un catalyseur prenant la forme d'un métal provenant du groupe 8, 9 ou 10 du système périodique des éléments et/ou de son oxyde métallique,
le composé formé étant un dérivé d'urée de formule générale (IV) : 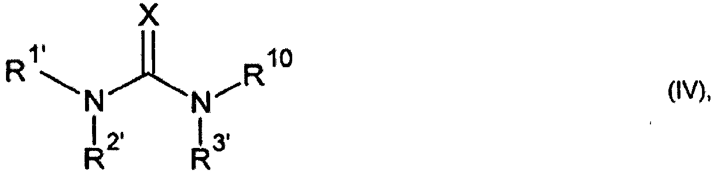 dans laquelle
-- R¹, R^{2'}, R^{3'}, indépendamment les uns des autres, représentent
--- R¹⁰,
--- une liaison covalente liée à un radical R¹⁰ relié au même atome d'azote du dérivé d'urée de sorte qu'une structure cyclique se forme à partir de l'atome d'azote et du radical R¹⁰,
--- H ou
--- un groupe alkyle en C₁ à C₂₀ linéaire ou ramifié, cycloalkyle en C₅ à C₂₀, aryle en C₅ à C₂₀, aryle en C₅ à C₂₀ substitué par un groupe alkyle en C₁ à C₂₀, qui peut être interrompu, respectivement, par un ou plusieurs atomes d'oxygène, atomes de soufre, et/ou atomes d'azote substitués, et/ou par un ou plusieurs groupes du type -C(O)O-,
-OC(O)-, -C(O)- et/ou -OC(O)O- et/ou qui peut être fonctionnalisé par un ou plusieurs groupes hydroxy et/ou groupes mercapto, et
-- R¹⁰ représente un groupe alkyle en C₁ à C₂₀ linéaire ou ramifié, cycloalkyle en C₅ à C₂₀ ou aryle en C₅ à C₂₀ substitué par un groupe alkyle en C₁ à C₂₀, qui peut être interrompu, respectivement, par un ou plusieurs atomes d'oxygène, atomes de soufre, et/ou atomes d'azote substitués, et/ou par un ou plusieurs groupes du type -C(O)O-, -OC(O)-, -C(O)- et/ou -OC(O)O- et
a) qui comprend au moins un autre groupe fonctionnel choisi dans la classe comprenant les groupes hydroxyle, les groupes mercapto, les groupes acide carboxylique, les groupes ester d'acide carboxylique, les groupes amide d'acide carboxylique, les groupes cétone, les groupes carbonate, les groupes époxy et les groupes carbamate, ou
b) forme alors une structure cyclique comprenant deux liaisons covalentes liées au même atome d'azote des composés de formules générales (III) ou (IV), une des deux liaisons covalentes étant fournie par l'un des radicaux R¹, R^{2'}, R^{3'}, et au moins un autre groupe fonctionnel, choisi dans la classe comprenant les groupes hydroxyle, les groupes mercapto, les groupes acide carboxylique, les groupes ester d'acide carboxylique, les groupes amide d'acide carboxylique, les groupes cétone, les groupes carbonate, les groupes époxy et les groupes carbamate, pouvant être présent dans le radical R^{10'}, et
-- X représente O ou S,

2. Procédé selon la revendication 1, **caractérisé en ce que** la réaction s'effectue en présence d'au moins une autre substance, choisie dans la classe comprenant l'ammoniac, l'azote, l'argon, l'hydrogène et/ou le monoxyde de carbone.

3. Procédé selon la revendication 2, **caractérisé en ce que** le rapport quantitatif entre l'autre substance et le réactif est de 0,1 à 2, en particulier de 0,5 à 1,5 et tout particulièrement de 0,8 à 1,3.

4. Procédé selon l'une des revendications précédentes, **caractérisé en ce que** la réaction s'effectue à une pression totale de 1 à 200 bar, en particulier 40 à 180 bar et tout particulièrement 60 à 140 bar et à une température de 100 °C à 300 °C, en particulier de 150 °C à 250 °C, en particulier de 180 °C à 240 °C.

5. Procédé selon l'une des revendications précédentes, **caractérisé en ce que** la réaction s'effectue pendant une durée de 1 minute à 100 heures, en particulier de 1 heure à 20 heures et tout particulièrement de 4 heures à 12 heures.

6. Procédé selon l'une des revendications précédentes, **caractérisé en ce que** le catalyseur comprend un matériau support.

7. Procédé selon la revendication 6, **caractérisé en ce que** le matériau support comprend une zéolithe, un aluminosilicate, un aluminophosphate, un oxyde métallique, un silicate, un silicate lamellaire, de l'oxyde d'aluminium, du dioxyde de silicium et/ou du carbone.

8. Procédé selon l'une des revendications précédentes, **caractérisé en ce qu'**au moins un groupe hydroxyle du réactif est présent sur le radical alkyle et non sur le radical aryle quand R¹⁰ représente un groupe aryle en C₅ à C₂₀ substitué par un groupe alkyle en C₁ à C₂₀.

9. Procédé selon l'une des revendications précédentes, **caractérisé en ce qu'**au moins un radical, en particulier au moins deux radicaux, en particulier au moins trois radicaux, parmi les radicaux R^{1'}, R^{2'} R^{3'}, a ou ont la signification du radical R¹⁰.

10. Procédé selon l'une des revendications précédentes, **caractérisé en ce que** le composé formé est choisi dans le groupe comprenant l'acide N-monoalkylcarboxylique-urée, l'acide N,N'-dialkylcarboxylique-urée, l'acide N,N,N'-trialkylcarboxylique-urée, l'acide N,N,N',N'-tétraalkylcarboxylique-urée.
